# EUROPEAN PATENT APPLICATION

(11) **EP 1 653 219 A1**
(43) Date of publication of application: **03.05.2006**
(21) Application number: 04256593.7
(22) Date of filing: 26.10.2004
(51) Int. Cl.: G01N 21/57, A61B 5/107

(54) **Method and apparatus for examining human hairs**

(71) Applicant: THE PROCTER & GAMBLE COMPANY, Cincinnati, Ohio 45202 (US)
(72) Inventor: Gummer, Christopher Lawrence, Chilworth Surrey GU4 8RR (GB); Baker, Paul Edmund, Silchester Hampshire RG7 2NJ (GB)
(74) Representative: Engisch, Gautier

(57) **Abstract**

Method and apparatus for examining human hairs (1) by means of a top illumination beam (2), in which a light beam (3) reflected from the hairs (1) is registered and used for an evaluation, comprising means for scanning at least part (W) of the width (4) of the number of human hairs (1) at least once in a line (5) with the top illumination beam (2), and a device (6) registering the reflected light beam (3) produced in the process continuously as proportional electrical signals (103), and a counter (71) counting the signals (103) as amount of the number of hairs (1), and an evaluator (72) evaluating the amount of the number of hairs (1) and displaying it via a display (32).

## Description

### FIELD OF INVENTION

The invention relates to a method and an apparatus for examining human hairs, according to the precharacterizing clause of Claim 1 and 8, respectively.

### BACKGROUND OF INVENTION

Methods and apparatus of this type are known in a wide range of embodiments, for example for examining the gloss of human hairs by means of a top illumination beam, in which a light beam reflected from the hairs is registered and used for an evaluation (e.g. see JP20116622A2).

The invention is based on the object of providing a method and an apparatus equivalent to the generic type, with which a simple, flexible, rapid, and accurate examination of human hairs is to be made possible.

### SUMMARY OF THE INVENTION

This object is achieved in that, in the method, at least part of the width of a number of human hairs is scanned at least once in a line by the top illumination beam, the reflected light beam produced in the process being registered and used for counting of the amount of the number of hairs and being evaluated and being displayed.

This object is achieved in that, the apparatus scans at least part of the width of the number of human hairs at least once in a line with the top illumination beam, the device registering the reflected light beam produced in the process continuously as proportional electrical signals, and a counter counting the signals as amount of the number of hairs, and an evaluator evaluating the amount of the number of hairs and displaying it via a display.

Advantageous developments of the invention emerge from the respective subclaims.

The method and the apparatus are very useful by a beauty shop or/and a point of sale or/and a laboratory.

This method/apparatus is used to quantify the amount of gray hair in the consumers hair or root line to tell them when to color.

This method/apparatus has the capability to measure tonal variation across the hair strands. This could be used to quantify natural hair colors and tones. As consumers constantly state that they want natural colored hair but nobody knows how to quantify this today. This method/apparatus also is used to quantify natural colored hair, so there is a better selection of hair colorants.

### BRIEF DESCRIPTION OF THE DRAWINGS

The invention will be described in more detail using a number of figures, in which:
- Fig. 1: shows part of a number of human hairs in a plan view;
- Fig. 2: shows the number of hairs according to Fig. 1 in a side view;
- Fig. 3: shows an enlarged illustration of a detail X1 from Fig. 1;
- Fig. 4: shows an enlarged illustration of a detail X2 from Fig. 1, but with another number of human hairs;
- Fig. 5: shows an enlarged illustration of the line 5 from Fig. 1;

- Fig. 6: shows an enlarged illustration of a detail Y1 from Fig. 5;
- Fig. 7: shows a part of a CCD array;
- Fig. 8: shows an enlarged illustration of a detail Y2 from Fig. 5; but with another number of human hairs;
- Fig. 9: shows a part of a CCD array as Fig. 7;
- Fig. 10: shows an enlarged illustration of a detail Y1 from Fig. 5 as Fig. 6;
- Fig. 11: shows electrical signals by a laser light sensor;
- Fig. 12: shows an enlarged illustration of a detail Y2 from Fig. 5 as Fig. 8;
- Fig. 13: shows electrical signals by a laser light sensor;
- Fig. 14: shows, in a side view, a hand-held bar scanner or reader during the examination of a number of hairs on a head;
- Fig. 15: shows a hand-held bar scanner or reader during the examination of a number of hairs on a head with a computer and a display;
- Fig. 16: shows a diagram with an amount of grey hair on a head and in a switch;
- Fig. 17: shows a diagram with a range of reflectance values from switches;
- Fig. 18: shows a diagram with a demonstration of lightness and tonal variation from root to tip on longer hair;
- Fig. 19: shows a diagram with a change in hair reflectance before and after coloring virgin and grey blended switches.

### DETAILED DESCRIPTION OF THE INVENTION

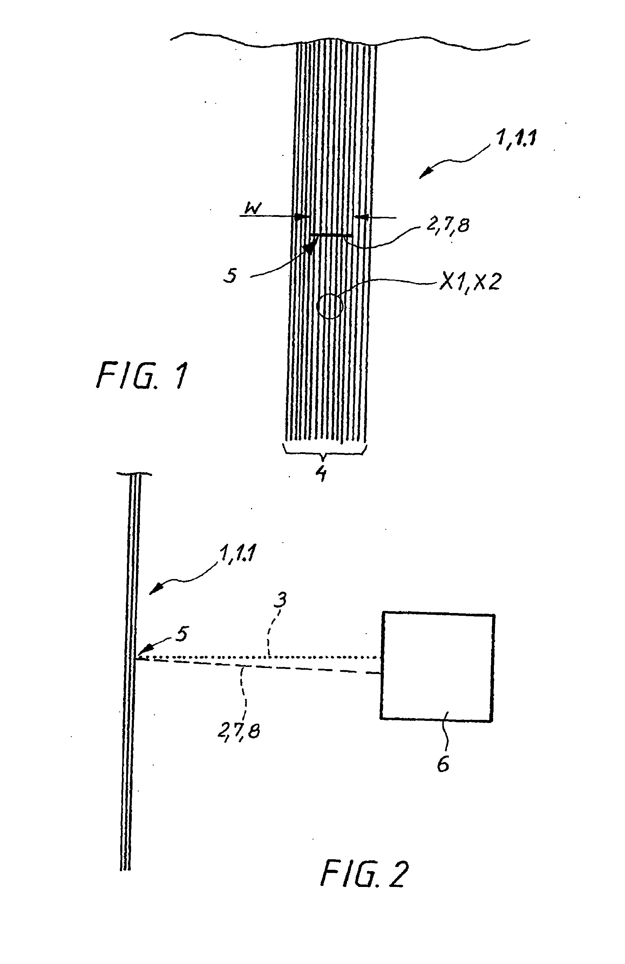

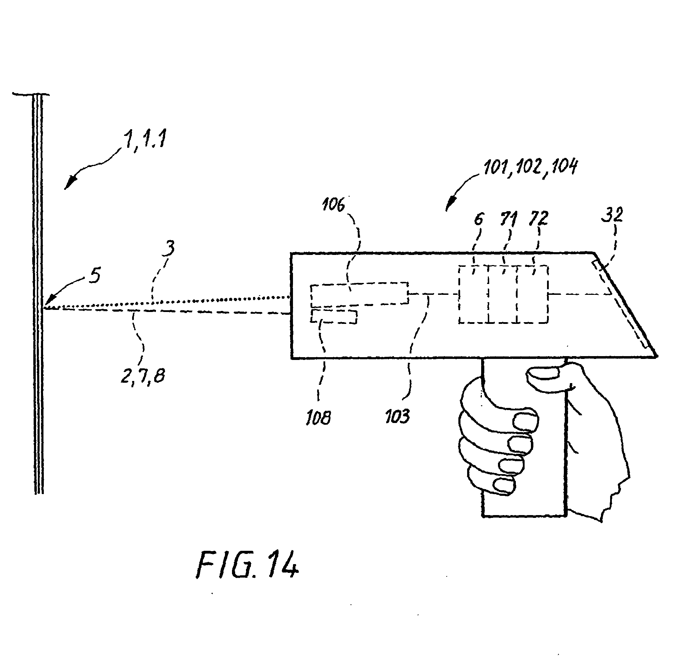

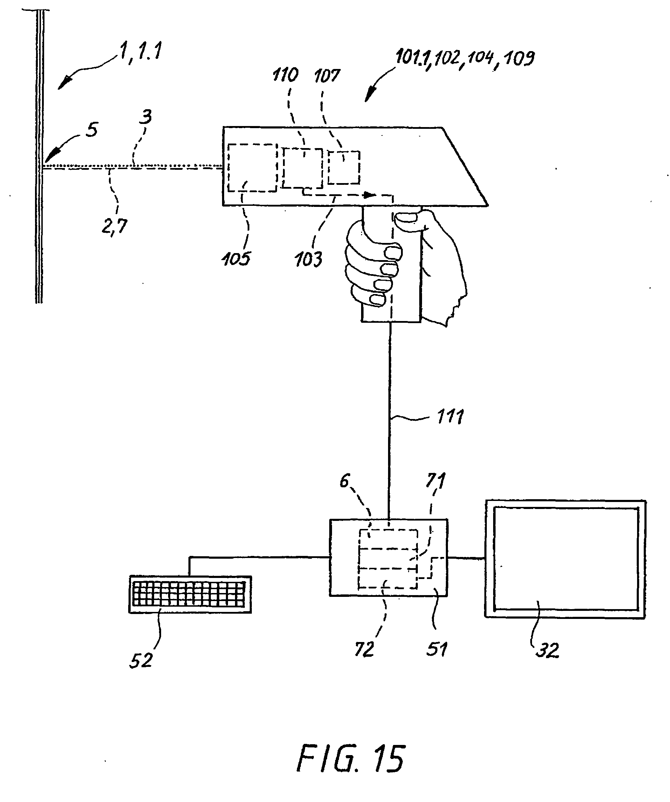

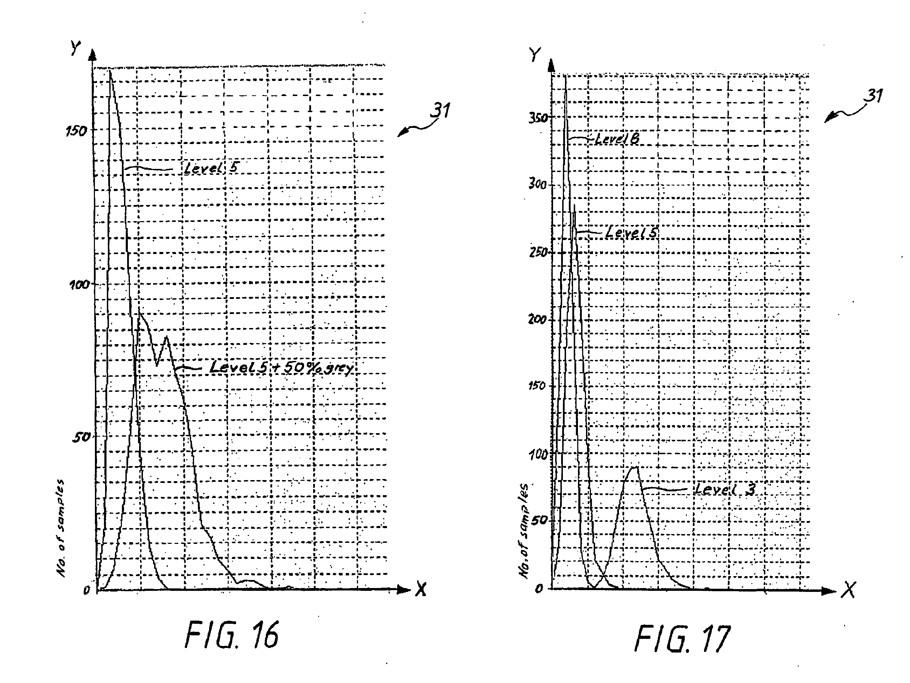

**Fig. 1** shows a method for examining a number of human hairs 1, for example a strand 1.1 of hair on a head or removed from head belonging to a person by means of a top illumination beam 2, in which a light beam 3 reflected from the hairs 1 is registered and is used for an evaluation. At least part W of the width 4 of the strand 1.1 of the number of hairs 1 is scanned at least once in a line 5 by the top illumination beam 2, the reflected light beam 3 produced in the process being registered (by a device 6 / **Fig. 2**) and used for counting (by a counter 71 / Figs 14 and 15) of the amount of the number of hairs 1 and evaluated (by an evaluator / Figs 14 and 15) and being displayed (by a display 32 / Figs 14 and 15). The display 32 is preferably carried out graphically, for example by means of a graph 31 (Figs 16-19).

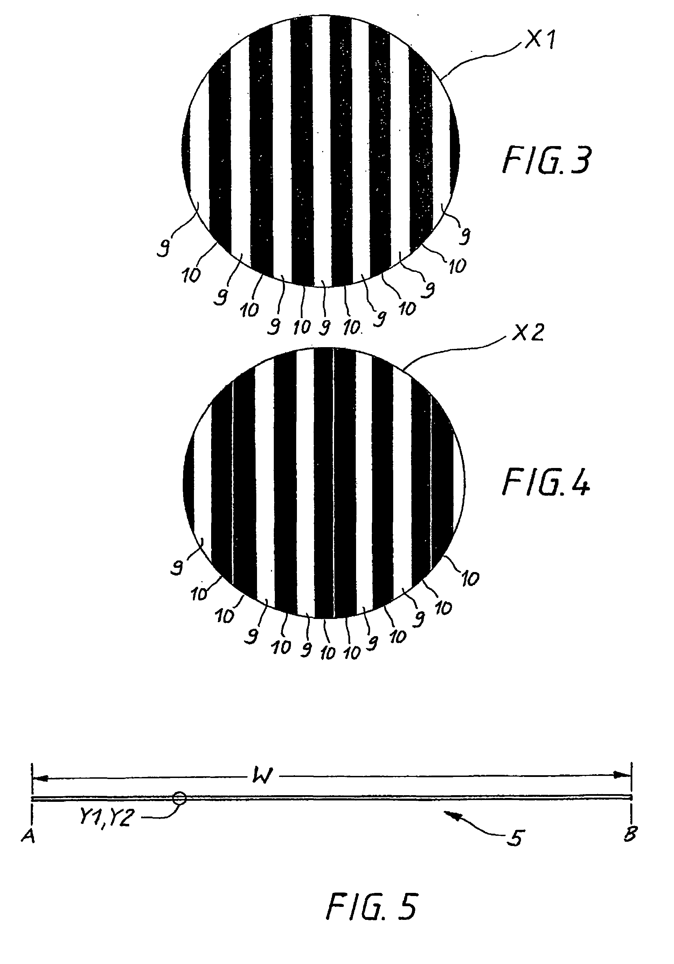

In an exemplary embodiment of the method, a proportion of grey hair of a number of hairs 1 of a strand 1.1 of hair on a head is determined, individual white hairs 9 and individual dark (or black or colored) hairs 10 (**Figs 3 and 4**) within the line 5 (Fig. 1) being scanned in the same way as when scanning a bar code. The data from the individual hairs 9, 10, registered by the device 6, are counted and evaluated by means of a computer 51 (Fig. 15) and a suitable software program and is displayed by a display 32 (Fig. 15). **Fig. 3** shows 50 % white hairs 9 and 50 % colored hairs 10 of a strand 1.1 of hair. With this method, a line or hair resolution of up to 3 µm is possible, which means that normal hair thicknesses of 0.05 to 0.07 mm can be registered in every case.

By means of a white top illumination beam 2 or a RGB (red-green-blue)-LED beam (for example by means of a CCD optics and array 106, Fig. 14), a color of the strand 1.1 of hair is determined by an evaluator 72 by means of a suitable software program , if appropriate the proportion of colored hairs 10 and the proportion of white hairs 9 being registered in relation of the total amount of the number of hairs 1 being registered, evaluated and displayed or/and a tonal variation of the number of hairs 1 is determined by the device 6 by quantifying natural hair colors and tones. The corresponding displays 32 can optionally also be provided numerically by stating the percentage proportions of colored hairs 10 and white hairs 9.

The strand 1.1 of hair is advantageously scanned at a distance directly on the head or removed from the head of hair of the person, the reflected light beam 3 with a wavelength in the visible range permitting optical monitoring of the region of the strand 1.1 of hair on the head registered.

**Fig. 4** shows 40 % white hairs 9 and 60 % colored hairs 10 of a strand 1.1 of hair.

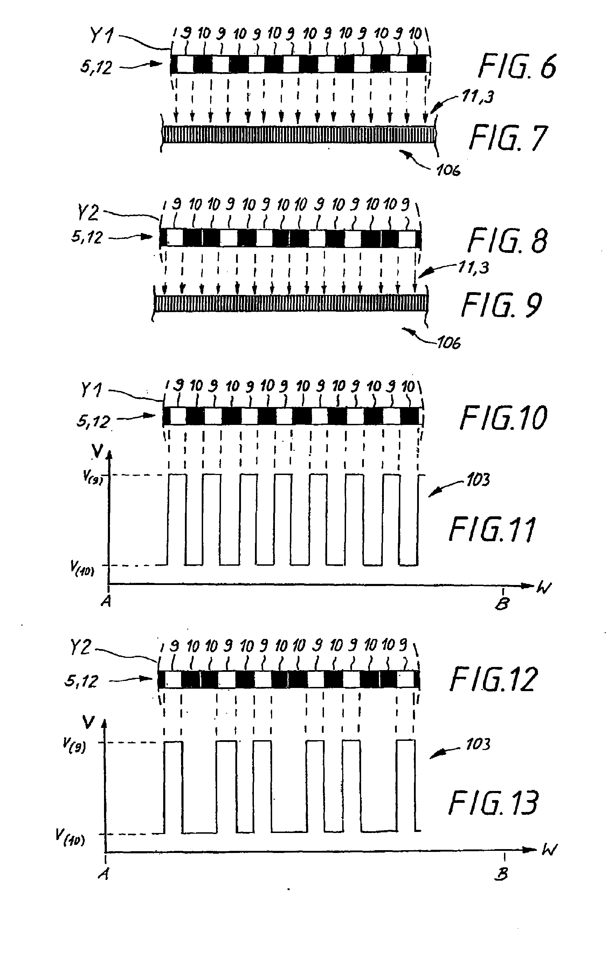

**Fig. 5** shows an enlarged illustration of the line 5 from Fig. 1. For example the scanning line 5 has a width W of 15 mm. Within this width W about 300 hairs are determined and so the result is an accurate evaluation and examination. By scanning with a CCD scanner the width W from A to B is an image 11 (**Figs 6 and 8**) of a surface 12 of hairs 1 on a CCD array 106 (**Figs 7 and 9**). By scanning with a laser scanner109 the width W of A to B being continuous electrical signals 103 (**Figs 11 and 13**) with values V from a reflected laser light beam 3 of a surface 12 (**Figs 10 and 12**) of hairs 1. The values V of white hairs 9 are higher than colored hairs 10, so there are different values V by white and colored hairs 9, 10. The image 11 of the hairs 1 is transferred to a row by CCD array/strip 106. The characteristics of the image 11 are determined interpreted, as by each individual photoelectric cell an electronic sample is provided, each line and gap by the number of neighboring cells, which determine black or white or color. Differently expressed to read in place of each line and each gap in consequence the CCD array takes up a picture of a very narrow part of the entire width W of the hairs 1, which it converts then into a decodable electrical signal 103.

**Fig. 14 and 15** show a first and a second apparatus 101, 101.1 for implementing the method for examining human hairs 1 or a strand 1.1 of hair on the head or removed from the head of a person by means of a top illumination beam 2, in which a light beam 3 reflected from the hairs 1 is registered and used for an evaluation, at least part of the width 4 of a number of human hairs 1 or the strand 1.1 of hair being scanned at least once in a line 5 by the top illumination beam 2, the reflected light beam 3 produced in the process being registered and used for counting of the amount of the number of hairs 1 and evaluated and being displayed. The Apparatus 101. 101.1 for examining human hairs 1 by means of a top illumination beam 2 , in which a light beam 3 reflected from the hairs 1 is registered and used for an evaluation, comprising:
means for scanning at least part (width W, Fig. 1) of the width 4 of the number of human hairs 1 at least once in a line 5 with the top illumination beam 2, and a device 6 registering the reflected light beam 3 produced in the process continuously as proportional electrical signals 103, and a counter 71 counting the signals 103 as amount of the number of hairs 1, and an evaluator 72 evaluating the amount of the number of hairs 1 and displaying it via a display 32.

By means a proportion of grey hairs of the number of hairs 1 is determined by registering individual white hairs 9 in relation of the total amount of the number of hairs 1 being registered (Figs 6-15).

By means a colored proportion of the number of hairs 1 is determined by registering individual colored hairs 10 in relation of the total amount of the number of hairs 1 being registered (Figs 6-15).

By means a tonal variation of the number of hairs 1 is determined by quantifying natural hair colors and tones (Figs 6-15).

The apparatus 101, 101.1 is provided with a bar scanner/reader 102 in a manner of a bar code scanner/reader, by means of which at least part (width W, Fig. 1) of the width 4 of the number of hairs 1 or the strand of hair 1.1 is scanned at least once in a line 5 by the top illumination beam 2, the device 6 registering the reflected light beam 3 produced in the process continuously as proportional electrical signals 103, and a counter 71 counting the signals 103 as amount of the number of hairs 1, and an evaluator 72 evaluating the amount of the number of hairs 1 and displaying it via a display 32.

Optical scanning systems have been developed for reading indicia such as barcode symbols appearing on labels or on the surfaces of articles. Typically, these systems include a laser scanning device (e.g. see US 4,896,026 A and WO 94/18642 A) or charge coupled device (CCD) scanner/reader (e.g. see US 5,869,840 A and US 6,581,838 B1) for reading barcodes. In general terms, a bar code scanner/reader 102 is an electronic-optical device for reading bar codes (in direct contact or at a distance), drawings, photographs and texts into a computer and scans an original line by line with the light beam and converts the (black/white) light values, grey steps or colors measured in the process into a sequence of byte values. These byte values are then processed further by a computer, for example with a graphics or OCR program.

Bar code CCD-scanners/readers are usually provided with an LED (660 nm) and a CCD-array with 2048 or more pixels, a resolution of less than 0.1 mm being achieved and approximately 100 scans/second being carried out. The maximum distance specified is approximately 130 mm. In the case of a laser scanner/reader, a substantially higher resolution is possible. Bar code CCD-scanners/readers are distinguished by the fact that the construction is very simple and they have a low weight. By comparison, laser scanners/readers are more complicated in construction, as a result of a rotating mirror arrangement, and have a higher weight.

The first apparatus 101 i.e. the bar scanner/reader 102 is provided as a hand-held device 104 in the manner of a hand-held bar code scanner or reader. The bar scanner/reader 102 can also be provided as a bench mounted unit. The cordless (CCD) bar scanner/reader 102 according to **Fig. 14** is provided with CCD optics 106 and a (white) LED radiation source 108. The LED light source 108 produces a top illumination beam 2 having a linear cross section 8 which corresponds to the width W (Figs 1 and 5) of the scanning line 5. The top illumination beam 2 and the reflected light beam 3 having approximately the same angle. The CCD optics 106 contain a CCD array/strip, which means that not only lines but also colors can be detected. Using a white top illumination beam 3, color analyses of a strand 1.1 of hair may be performed via the CCD array 106. The electrical signals 103 from the reflected light beam 3 registered by the CCD optics 106 are evaluated by the evaluator 72 and displayed by a display 32. The device 6 is provided with a programmed microcomputer capable of transforming the electrical signals 103 into the display 32, and means for displaying the display 32. Provision can optionally also be made for the data registered to be transmitted in a wire-free manner to a computer 51 (Fig. 15), which performs a further evaluation of the data and a corresponding display from the evaluation via the display 32.

The second apparatus 101.1 i.e. the hand-held device 104 according to **Fig. 15** has a laser scanner/reader 109 with a laser diode 107, the top illumination (laser light) beam 2 being point-like 7 in cross section, which scans a width W (Fig. 5) of the width 4 of the strand of hair in a line 5 via a rotating mirror arrangement 105 and, at the same time, registers the reflected light beam 3 via a light sensor 110. The top illumination beam 2 and the reflected light beam 3 having the same angle. The electrical signals 103 registered by the light sensor 110 are supplied via an electric lead 111 to a computer 51 which, by means of a device 6, a counter 71 and a evaluator 72 evaluates the electrical signals 103 and displays them via a display 32. The computer 51 is provided with a programmed evaluator 72 capable of transforming the electrical signals 103 into the display 32, and means for displaying the display 32. At least the evaluator 72 is a part of a computer program. The electric lead 111 can be used at the same time for the power supply of the laser scanner/reader 109. A keyboard 52 is provided for the operation of the computer 51. Another way for transferring the electrical signals 103 by an electric lead 111 is realized by a wire-less device (transmitter and receiver).

**Figs 16 - 19** show diagrams of reflectance values. Levels 3,5,8 are standard hair colours on a scale of 1-14. The scanner 101,101.1,102,104,109 would need to be able to tell the difference between these levels. Even though they are different colours of hair from black to white, the scanner can tell the difference due to the different levels of reflectivity of the incident light source. The x - axis is the reflectance value and the y - axis is the cumulative reflectance value (ie. the number of times that reflectance value occurs hence the somewhat bell shaped curves).

When the scanner is used we first get a graph of x-axis as the sample point from the hair sample along a line and the y-axis is the value of reflectance at that point. As a parallel, it would give the y-axis at that point. We then gather all the reflectance values. This is then turned into the cumulative reflectance graph outlined above.

**Fig. 16** shows a diagram with an amount of grey hair on a head and in a switch. The reflectance levels for level 5 + 50% grey hair are bi-modal and shifted to the right. The bi-modal distribution is expected from the dark (brown) hairs 10 and white (grey) hairs 9. However, the shift to the right suggests that the overall light reflectance, due to scatter, is now greater and the dark hairs 10 no longer overlap the original level 5 hairs. In consumer terms this should make grey hair appear lighter than just the average between grey and brown hair i.e., the grey hair looks worse than it is. The method is already sensitive to 30 % grey switches without additional data interpretation. The double peak of the graph "level 5 + 50 % grey" is the result of adding white hairs 9 to the original colour. The left hand peak is the cumulative reflectance graph from the original hair colour. The right hand peak is the same original colour but now with 50% of the hairs replaced by white hairs as would occur in grey hair. Also, when you colour grey hair the right hand curve will now be shifted to the left - hence a double peak representing before and after colouring.

**Fig. 17** shows a diagram with a range of reflectance values from switches. The reflectance plots show a shift to the right with increasing lightness (levels 3,5,8). In addition the width of the plot increases suggesting that light hair may appear lighter than its true color.

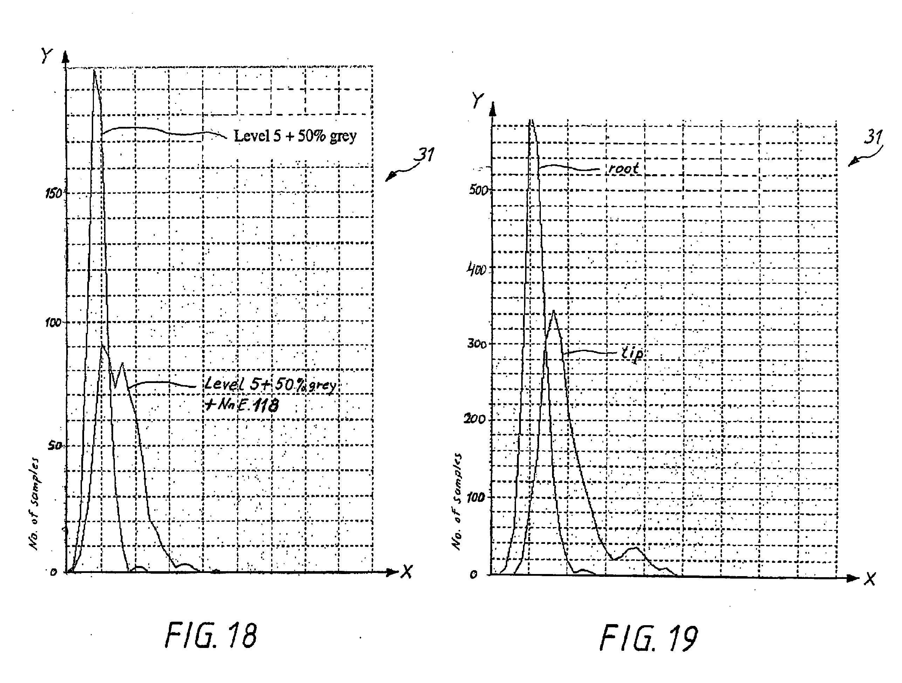

**Fig. 18** shows a diagram with a demonstration of lightness and tonal variation from root to tip on longer hair. An addition of an agent (Nice n Easy 118 to level 5+50 % grey hair) shows both slight darkening (shift to the left) and a more uniform reflectance (narrower peak). Importantly the grey peak has been removed but the width of the colored hair peak is similar to that from virgin level 5 hair. This indicates that it is retaining much of the natural variation in reflectance of normal hair after coloring.

**Fig. 19** shows a diagram with a change in hair reflectance before and after coloring virgin and grey blended switches. This single sample measured on head shows a narrower reflectance distribution at the scalp (roots) compared with the tips. The shift to the right also shows the tips to be lighter than the roots. Hair was measured at the scalp and at 30 mm distance from the scalp should change in lightness.

### List of designations:

- 51: A number of hairs
- 1.1: Strand of hair
- 2: Top illumination beam
- 3: Reflected light beam
- 4: Width (of a strand of hair or of a number of hairs)
- 5: Line
- 6: Device
- 7: Point-like cross section
- 8: Linear cross section
- 9: White hair
- 1: Colored (black/dark) hair
- 2: Image
- 3: Surface
- 101: Graph
- 102: Display
- 31: Computer
- 52: Keyboard

- 10: counter
- 11: evaluator
- 1: Apparatus
- 2: Bar scanner/reader
- 3: Electrical signal
- 4: Hand-held device
- 5: Mirror arrangement
- 6: CCD optics/array
- 7: Laser diode
- 8: LED
- 9: Laser scanner/reader
- 10: Light sensor
- 11: Electric lead

- W: Width of scanning field
- V: Value

## Claims

1. Method for examining human hairs by means of a top illumination beam, in which a light beam reflected from the hairs is registered and used for an evaluation, **characterized in that** at least part (W) of the width (4) of a number of human hairs (1) is scanned at least once in a line (5) by the top illumination beam (2), the reflected light beam (3) produced in the process being registered and used for counting of the amount of the number of hairs (1) and being evaluated and being displayed.

2. Method according to Claim 1, **characterized in that** a proportion of grey hairs of the number of hairs (1) is determined by registering individual white hairs (9) in relation of the total amount of the number of hairs (1) being registered.

3. Method according to Claim 1, **characterized in that** a colored proportion of the number of hairs (1) is determined by registering individual colored hairs (10) in relation of the total amount of the number of hairs (1) being registered.

4. Method according to Claim 1, **characterized in that** a tonal variation of the number of hairs (1) is determined by quantifying natural hair colors and tones.

5. Method according to Claim 1, **characterized in that** the number of hairs (1) are a strand (1.1) of hair.

6. Method according to Claim 1, **characterized in that** the number of hairs (1) is scanned directly on a head of hair belonging to a person.

7. Method according to Claim 1, **characterized in that** the number of hairs (1) are scanned removed from a head belonging to a person.

8. Apparatus for examining human hairs by means of a top illumination beam, in which a light beam reflected from the hairs is registered and used for an evaluation, comprising:
means for scanning at least part (W) of the width (4) of the number of human hairs (1) at least once in a line (5) with the top illumination beam (2), and a device (6) registering the reflected light beam (3) produced in the process continuously as proportional electrical signals (103), and a counter (71) counting the signals (103) as amount of the number of hairs (1), and an evaluator (72) evaluating the amount of the number of hairs (1) and displaying it via a display (32).

9. Apparatus according to Claim 8, **characterized in that** the apparatus (101) scans in the manner of a bar code scanner/reader (102).

10. Apparatus according to Claim 9, **characterized in that** the bar code scanner/reader (102) is provided as a hand-held device (104) or is provided as a bench mounted unit.

11. Apparatus according to Claim 8, **characterized in that** the top illumination beam is point-like (7) in cross section (2), scans the width (4) of the strand of hair in a line (5) via a rotating mirror arrangement (105) and registers the reflected light beam (3) at the same time.

12. Apparatus according to Claim 11, **characterized in that** the top illumination beam (2) is produced by a laser diode (107).

13. Apparatus according to Claim 8, **characterized in that** the top illumination beam (2) is linear (8) in cross section and irradiates at least part of the width (4) of the strand of hair, CCD optics (106) registering the reflected light beam (3).

14. Apparatus according to Claim 13, **characterized in that** the top illumination beam (2) is produced by a LED (108).

15. Apparatus according to Claim 8, **characterized in that** the top illumination beam (2) has a wavelength in the visible range.

16. Apparatus according to Claim 8, **characterized in that** white light is provided as the top illumination beam (2).

17. Apparatus according to Claim 8, **characterized in that** a proportion of grey hairs of the number of hairs (1) is determined by the evaluator (72) by registering individual white hairs (9) in relation of the total amount of the number of hairs (1) being registered.

18. Apparatus according to Claim 8, **characterized in that** a colored proportion of the number of hairs (1) is determined by the evaluator (72) by registering individual colored hairs (10) in relation of the total amount of the number of hairs (1) being registered.

19. Apparatus according to Claim 8, **characterized in that** a tonal variation of the number of hairs (1) is determined by the evaluator (72) by quantifying natural hair colors and tones.

20. Apparatus according to Claim 8, **characterized in that** at least the evaluator (72) is a part of a computer program.
